# EUROPEAN PATENT APPLICATION

(11) **EP 1 923 668 A1**
(43) Date of publication of application: **21.05.2008**
(21) Application number: 07022217.9
(22) Date of filing: 15.11.2007
(51) Int. Cl.: G01D 1/18, G01N 21/35

(54) **Method of processing an analog sensor signal in a gas sensor arrangement and measured value processing device**

(30) Priority: 16.11.2006 DE 102006054164
(71) Applicant: Tyco Electronics Raychem GmbH, 85521 Ottobrunn (DE)
(72) Inventor: Buchberger, Ludwig, 85757 Karlsfeld (DE); Frodl, Robert, 81825 Munich (DE)
(74) Representative: Grünecker, Kinkeldey, Stockmair & Schwanhäusser Anwaltssozietät

(57) **Abstract**

This invention concerns a method of processing an analog sensor signal, wherein the analog sensor signal, which is fed into a first input of an operational amplifier, is amplified by the operational amplifier. The amplified analog sensor signal is measured and compared with a threshold value. A direct voltage is generated depending on a difference between the amplified analog sensor signal and the threshold value, and a difference signal is formed from the analog sensor signal and the direct voltage. The difference signal is amplified and output as the output signal.

## Description

This invention refers to a method of processing an analog sensor signal; the method using an operational amplifier.

In particular, this invention refers to such a method for use in a gas sensor arrangement, to an associated gas sensor arrangement and to a measured value processing device.

Known gas sensor arrangements include a radiation-emitting radiation source, a gas measurement space, which can be filled with a measurement gas which includes at least one analyte to be measured, and at least one radiation-detecting detector device, which generates an output signal which depends on the absence and/or concentration of the analyte. Such gas sensor arrangements for proving the presence of a wide variety of analytes, e.g. carbon dioxide or methane, are known. Traditional gas sensors are based on the property of many polar gases that they absorb radiation in the infrared wavelength range. The IR light is capable of shifting the molecules into excited states by exciting rotation and vibration oscillations, by interacting with the dipole moment of the polar molecule. In this way, the heat energy of the IR light is transferred to the gas, and in the same way the intensity of an IR beam passing through the gas volume is reduced. Corresponding to the excitation states, the absorption occurs at a wavelength which is characteristic of the relevant gas, e.g. in the case of CO₂ at 4.25 µm.

In particular, today carbon dioxide detection is gaining increasing importance in many application fields. For instance, in the motor vehicle field, carbon dioxide detection can be used for monitoring the CO₂ content of the interior air to increase energy efficiency for heating and air-conditioning, to cause a fresh airflow via an appropriate ventilator flap drive only when required, i.e. in the case of increased CO₂ concentration. Also, modern motor vehicle air-conditioning systems are based on CO₂ as the coolant, so that CO₂ gas sensors in the motor vehicle field can carry out a monitoring function in relation to escaping CO₂ in the case of any defects. Particularly in the motor vehicle field, gas sensors must fulfill the highest requirements for robustness, reliability and miniaturizability. Additionally, for safety applications, the response time of the sensor must not exceed specified limits.

In German patent application DE102005032722, a gas sensor arrangement and a measurement method with early warning are described. In particular, this application refers to the radiation source which emits the radiation in the form of pulses. Also, German patent application DE102006019705.4 refers to a method of processing time-discrete measured values, the course of which over time can be described by means of a time function. The method according to this application uses a measured value filter to achieve a desired transient response.

In many gas sensor arrangements, as the detector device to analyze the IR radiation, so-called pyrosensors are used. The analog sensor signal which is output by such a pyrosensor has, depending on the measurement, a high offset voltage and only a small amplitude. For further processing and analysis of the signal, it is helpful to remove this offset voltage, but in this case the amplitude of the analog sensor signal should be amplified.

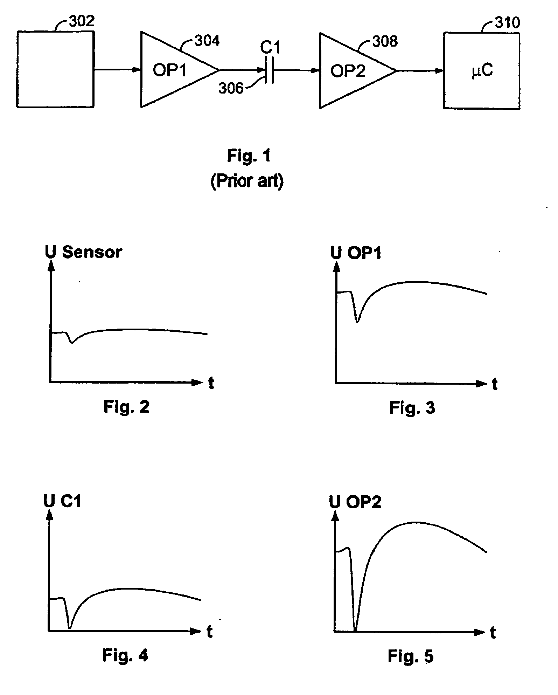

For instance, in the case of known arrangements, as shown in Fig. 1, the analog signal which the sensor 302 outputs is amplified in an operational amplifier 304. The offset voltage of this amplified signal is then removed by a capacitor 306, before the now cleaned analog signal is amplified in another operational amplifier 308. This amplified analog signal, without the offset voltage, is then further processed in a microcontroller 310.

The four curves in Figs. 2 to 4 show how the analog sensor signal is processed using the arrangement from Fig. 1. It can be seen that the amplitude of the signal which is output by the detector in Fig. 2 is only very small, and that it carries a large direct voltage part. The operational amplifier 304 amplifies the weak signal, but also the direct voltage part, so that the amplified signal possibly leaves the dynamic range of the subsequent analysis. The capacitor 306 blocks the direct voltage part out, see Fig. 4, and the operational amplifier 308 amplifies this cleaned signal until it can be measured well, see Fig. 5.

However, the coupling capacitor 306 is very temperature-dependent, which can create difficulties in the motor vehicle field, since a specified operating temperature cannot be ensured. In the temperature range from -40°C to +85°C, for instance, the capacitor changes its high capacitance, and it is also unstable in the long term. This coupling capacitor 306, because of its size, cannot easily be integrated on modules, and is therefore resource-intensive and expensive to construct.

The object on which this invention is based is therefore to give an improved method of processing analog sensor signals, which makes temperature-independent, fast and robust direct voltage suppression possible.

This object is achieved by the subject of the independent claims. Advantageous further developments of this invention are the subject of the dependent claims.

This invention is based on the idea that when the pure analog sensor signal is amplified without offset voltage, improved temperature behavior occurs if the offset voltage can be removed without using a capacitor. For this purpose, the output signal of the operational amplifier is fed back to minimize the offset.

According to the invention, over the whole voltage range a simple, temperature-independent, linear control is achieved. This makes simpler production of the component possible, and resource-intensive calibration is unnecessary.

The use of resistors according to the invention simplifies miniaturization of the gas sensor arrangement. The result is also a cost saving compared with the known use of a large capacitor.

The solution according to the invention also has the advantage that more precise measurements are made possible by the better signal resolution.

The advantageous properties of the measured value processing according to the invention can be exploited, in particular, in the case of gas sensor arrangements which are used for detection of carbon dioxide, e.g. in the motor vehicle field, both for monitoring for CO₂ escaping from leaks and for checking the air quality in the passenger compartment. Obviously, the principles according to the invention can also be used in relation to detection of any other gases, and are important for all sensors where a measurement signal with an unreliably high direct voltage part is to be analyzed.

The invention is explained in more detail below, on the basis of the advantageous versions which are shown in the attached drawings. Similar or corresponding details of the subject according to the invention are given the same reference symbols.
- Fig. 1: shows a circuit diagram of a known measured value processing device;
- Fig. 2: shows a first course of the signal over time in the arrangement from Fig. 1;
- Fig. 3: shows another course of the signal over time in the arrangement from Fig. 1;
- Fig. 4: shows another course of the signal over time in the arrangement from Fig. 1;
- Fig. 5: shows another course of the signal over time in the arrangement from Fig. 1;
- Fig. 6: shows a schematic representation of a gas sensor unit according to this invention;
- Fig. 7: shows a possible advantageous circuit diagram of the measured value processing device according to this invention;
- Fig.8: shows a table of resistance values according to one embodiment of this invention;
- Fig. 9: shows a graph of a correction voltage depending on a total resistance according to the table in Fig. 8;
- Fig. 10: shows another possible circuit diagram of the measured value processing device according to this invention;
- Fig. 11: shows a table of resistance values according to another embodiment of this invention;
- Fig. 12: shows a graph of a correction voltage depending on a total resistance according to the table in Fig. 11;
- Fig. 13: shows a table of resistance values according to another embodiment of this invention;
- Fig. 14: shows a graph of a correction voltage depending on a total resistance according to the table in Fig. 13.

The structure of the gas sensor arrangement according to the invention and the operation of the method according to the invention for amplifying the analog sensor signal will be explained in more detail below with reference to the figures.

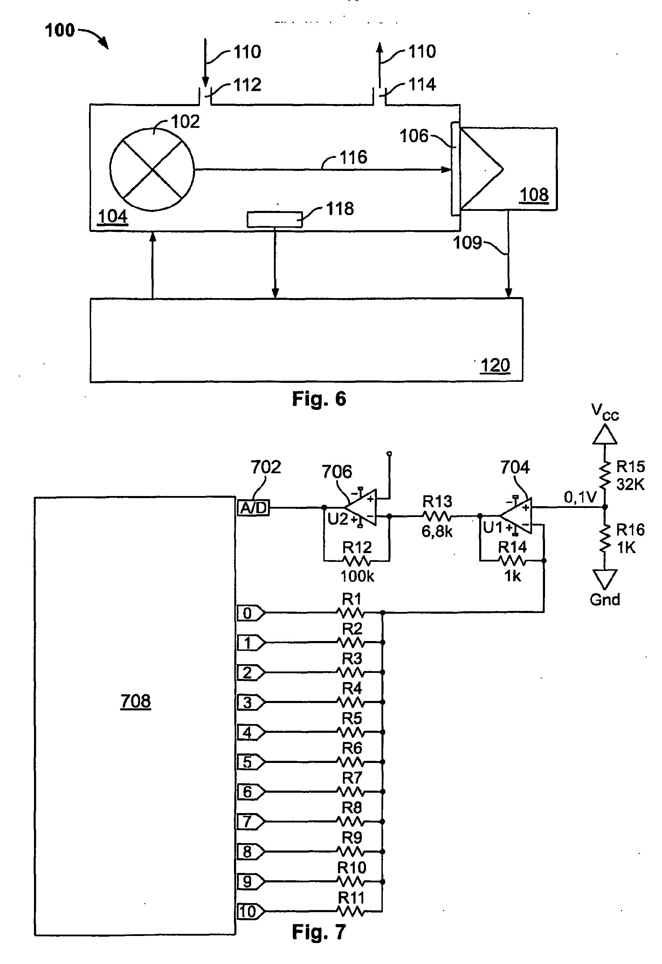

As shown in Fig. 6, a detector unit 108 captures a gas concentration, which here can be taken approximately as a Heaviside function. Obviously, however, the input signal for the detector unit 108 does not specifically have to be a gas concentration, but the output signal of any sensor can be processed according to the principles of this invention. The detector unit 108 supplies a time-discrete detector signal 109, which has an offset.

As shown in this figure, the gas sensor arrangement 100 according to the invention also includes a radiation source 102, here a broadband infrared radiation source. In principle, the shown gas sensor arrangement 100 is a so-called NDIR (non-dispersive infrared) sensor. The essential components, in addition to the infrared radiation source 102, are the gas measurement space 104, a wavelength filter 106 and an infrared detector as the detector unit 108. The temperature can optionally be measured by a temperature sensor 118. The measurement gas 110, which is to be checked for the gas component to be detected, is pumped into the gas measurement space 104 or diffused into it, which is symbolized by the inlets and outlets 112, 114. As explained above, the presence and/or concentration of the sought gas can be determined electro-optically via the absorption of a specific wavelength in the infrared range.

The emitted infrared radiation 116 is fed through the gas measurement space 104 into the detector unit 108. At the detector unit 108, an optical filter, which only lets through the wavelength range in which the gas molecules to be detected absorb, is arranged. Other gas molecules normally absorb no light at this specific wavelength, and therefore do not affect the quantity of radiation which reaches the detector unit 108. As the detector unit, all suitable infrared detectors can be used, and the signal processing method according to the invention can be adapted to the appropriate detector type.

For instance, the detector can be a pyroelement, an infrared thermopile or a photodiode. The suitable detector in each case should be chosen according to the requirements in each case. The photodiode has the advantage of being a comparatively inexpensive component, whereas the thermopile detector has the advantage of an especially high, even absorption of radiation in the selected spectral range. Finally, pyroelectrical sensors have the advantage of very high sensitivity and the possibility of miniaturized production.

The infrared signal is pulsed by the radiation source 102, to be able to filter out thermal background signals from the desired signal. Thus the measured values which the detector unit supplies are present in the form of time-discrete values, which essentially satisfy an exponential function.

A controller 120, on the one hand, activates the radiation source 102, and on the other hand receives the detector signals of the detector unit 108 and processes them further according to the principles of this invention. In particular, the controller includes a filter unit, which does the conversion of the detector signal 109 into an amplified output signal without offset.

For most application cases in gas sensors, not only the final value of the signal, but above all, the gradient of the signal is the important magnitude.

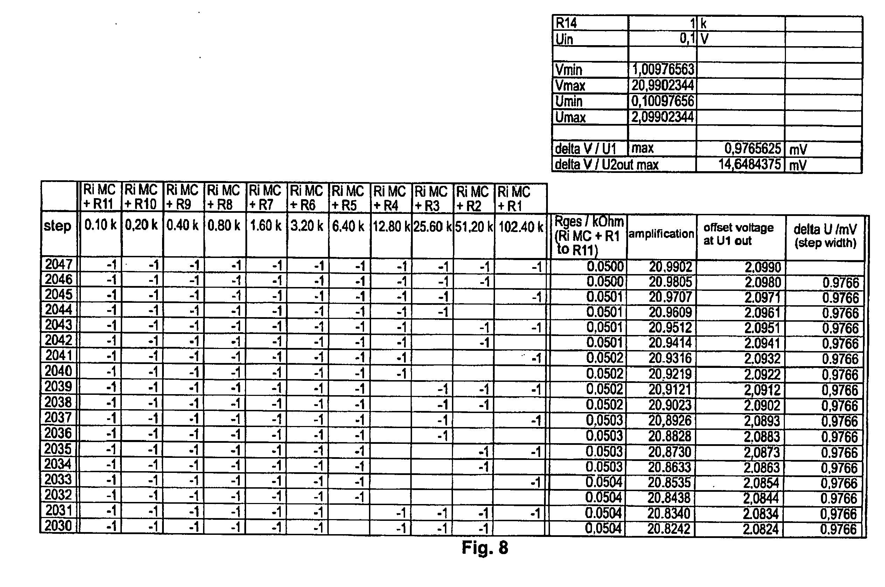

As shown in Fig. 7, the analog sensor signal is fed into one input of the operational amplifier 706. The output signal of the operational amplifier is connected to the analog/digital converter 702, which is controlled by the controller 708. The controller 708 also controls its own switch outputs 0 to 10, which can be switched between the "negative supply voltage", "open" and "positive supply voltage" states. Multiple resistors R1 to R11 are switched in different combinations between the switch outputs 0 to 10 of the microcontroller 708, each of which can have one of the three above-mentioned states, and the negative input of the differential amplifier 704. The output of this differential amplifier 704 is fed through a resistor R13 into the negative input of the differential amplifier 706, which then amplifies the difference signal between the analog sensor signal and the offset, which was fed into the negative input.

Below, the method of functioning of the measured value processing device according to the invention is described.

In the amplifier 706, a microcontroller-controlled voltage is subtracted from the analog sensor signal, and the difference is simultaneously amplified, to generate an output signal. The microcontroller 708 measures the output signal, for which purpose an analog/digital converter 702, which converts the amplified analog output signal of the operational amplifier 706 into a digital input signal for the microcontroller 708, is used. The microcontroller 708 decides about a signal correction on the basis of the digital input signal and the specified threshold value.

This threshold value can be determined by the operating voltage of the microcontroller 708, but other factors may also play a part. If the output signal of the differential amplifier 706 is not in the desired range, it is counteracted with a direct voltage, to suppress the offset signal. The result is amplification of the pure analog signal without offset voltage, as can be seen in the course over time in Fig. 5. In this way the amplified analog sensor signal is put into the active measurement range. In the invention, the analog sensor signal corresponds to the signal in Fig. 3, and the output signal of the operational amplifier 706 corresponds to the signal in Fig. 5.

The direct voltage is generated as follows: the resistors R15 and R16 define the voltage value of the positive input signal of the operational amplifier 704; in the embodiment of Fig. 7, this voltage is set at 0.1 V. The negative input signal of the operational amplifier 704 is given by the combination of resistors R1 to R11. Each of these resistors R1 to R11 can be connected to the switchable digital outputs 0 to 10 of the microcontroller 708 at one of three voltage values, namely positive or negative operating voltage or no voltage, i.e. open.

Next, the values of the resistances between the negative operating voltage and the voltage at the negative input of the operational amplifier 704, and the values of the resistances between the positive operating voltage and the voltage at the negative input of the operational amplifier 704, can be switched individually or in parallel, the microcontroller 708 controlling the switching. A parallel circuit of at least two resistors reduces the total resistance value. In this way, via relatively few resistors, many different voltage values can be reached, namely 2ⁿ or 3ⁿ combinations. This makes possible a variable setting of the value of the direct voltage which is applied to the negative input of the amplifier 704. In this way, dynamic offset compensation in the amplifier branch is achieved.

For instance, if the microcontroller 708 in the course of time detects that the voltage of the output signal is reaching its maximum operating voltage - the signal is fed into the analog/digital converter 702, which is integrated in the microcontroller 708, and which tolerates only a specified maximum voltage - the resistors R1 to R1 are switched so that a greater constant voltage is generated. The differential amplifier 706 forms the difference of the two signals, which then, in this case, has become smaller, and simultaneously amplifies the result, to achieve a better signal analysis. The signal output of the amplifier 706 can now be compared with the signal "U OP2" in Fig. 5.

During the measured value recording of the course of time for a radiation pulse, the resistance values must be constant. After the measurement, switching takes place if necessary, and a new measurement is then started.

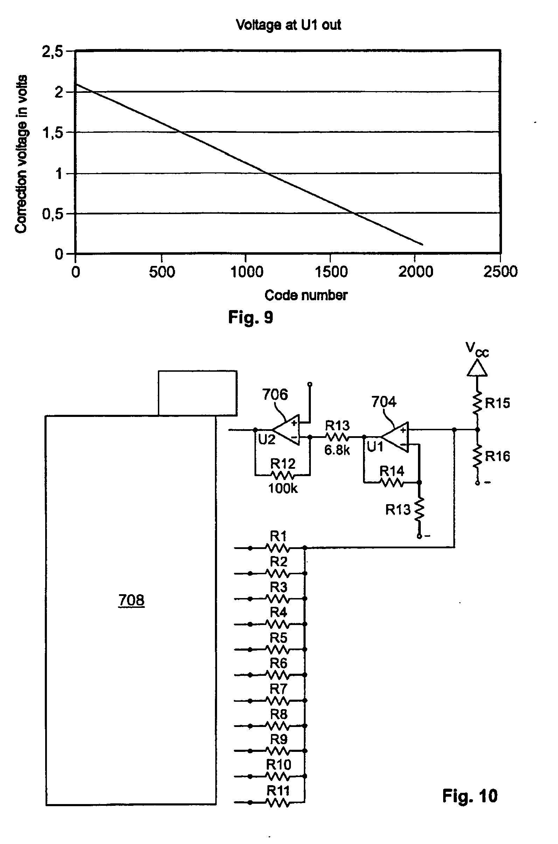

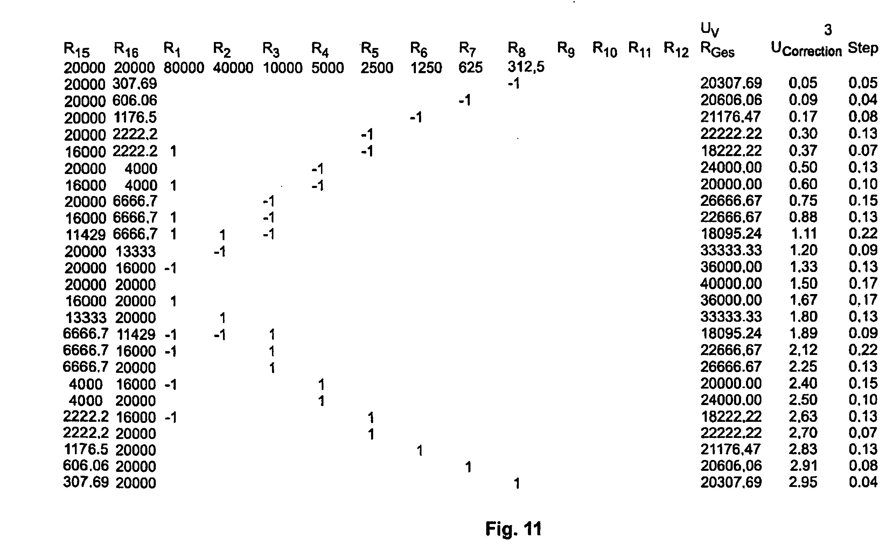

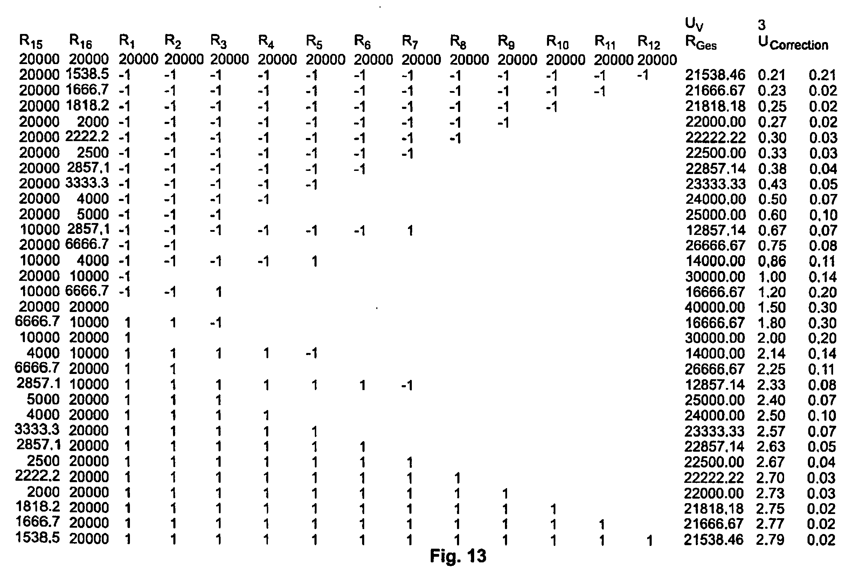

With reference to Figs. 7 to 14, two embodiments of this invention are shown in more detail, their circuit diagrams being in Figs. 7 (first embodiment) and 10 (second embodiment). Additionally, Figs. 8 and 9 belong to the first embodiment, Figs. 11 to 14 give more information about the second embodiment. In the tables of Figs. 8, 11 and 13, the used values of the resistors R1 to R11 or R12 are shown. A "1" means that the resistor is connected to the operating voltage, whereas a "-1" shows that the resistor has been switched to the negative operating voltage. If no value is given in the relevant column, the switch within the microcontroller 708 is open. The second row gives the values of the relevant resistor in ohms. From Figs. 8 and 11, it can be seen that the resistors all have different values, and in Fig. 11 only digital outputs 0 to 7 are populated with resistors. In Fig. 13, on the other hand, resistors R1 to R12, which all have the same value, 20000 Ω, are given.

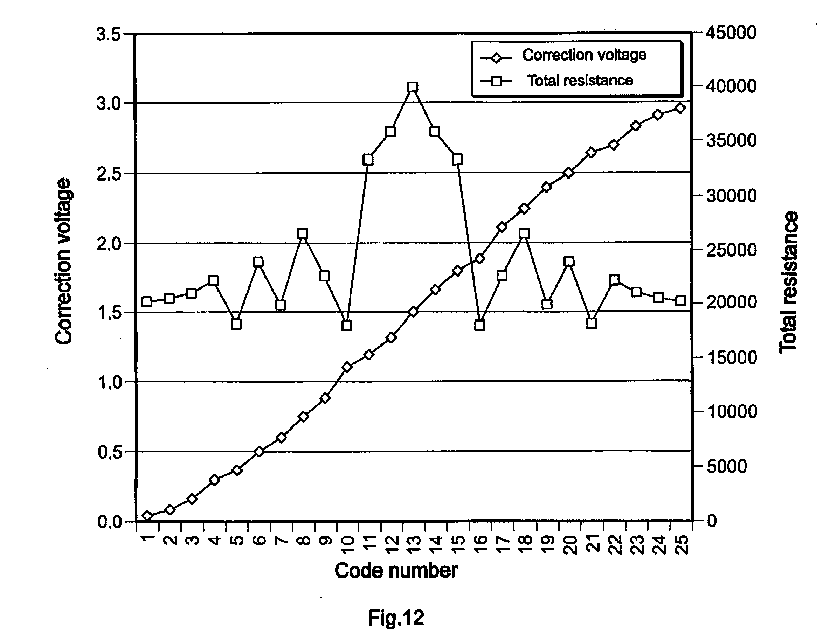

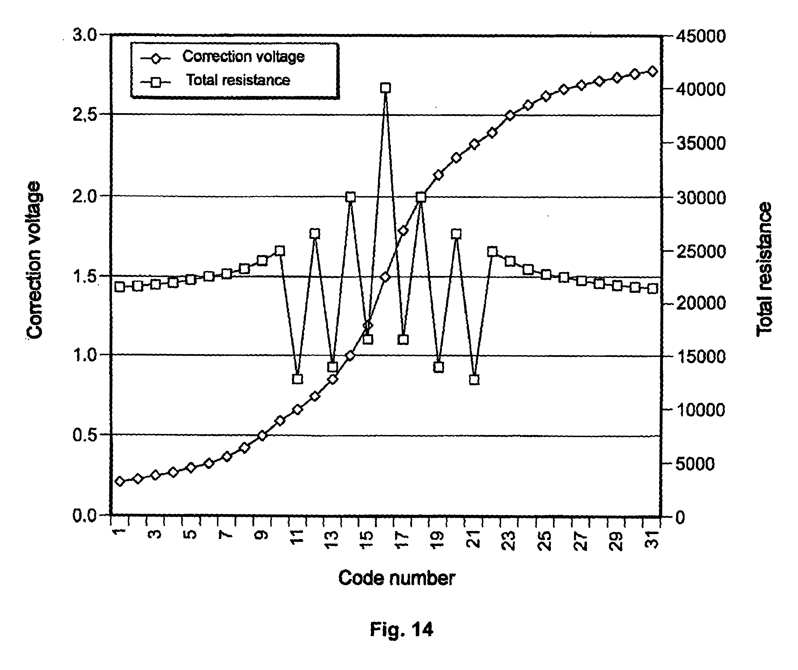

In Fig. 9, the correction voltage is shown against the code number; in Figs. 12 and 14, the total resistance compared with the correction voltage is shown. Code numbers can also be assigned to the various combinations of resistors, and they can be found here on the x-axis. Each code number stands for a specified combination of resistors, and they can be found in the rows of the tables of Figs. 8, 11 and 13. The microcontroller 708 stores the various combinations of resistors under these code numbers.

It is pointed out that the embodiments in the tables in Figs. 8, 11 and 13 are only partly occupied, to ensure a clear representation; more intermediate steps are possible. In addition to as small a step width as possible, as constant as possible a total resistance of the arrangement is desirable. Additionally, the voltage difference between symmetrical code pairs should be approximately equal, which is achieved by not all resistors R1 to R11 being connected to positive or negative operating voltage, but many of the digital inputs 0 to 10 of the microcontroller 708 being "open". That has the further advantage that less memory space is required for a code assignment table in the microcontroller 708. In Fig. 8, only the first rows of the table are listed; the other values can be calculated immediately. In principle, a count down takes place in a Boolean manner.

It has also been shown that a linear course of the offset correction is possible, if it is taken into account that the digital outputs 0 to 10 of the microcontroller 708 have a significant internal resistance.

The amplification of the analog sensor signal without offset will be clarified further using Figs. 2 to 5. The output signal of the pyrosensor is given in Fig. 2. As is made clear in Fig. 3, this signal is amplified, so that the amplitude of the signal is amplified, but the offset is also magnified. This signal in Fig. 3 is the amplified analog sensor signal. The output signal of the operational amplifier 706 is then shown in Fig. 5, in which it can clearly be seen that the offset has been reduced and the amplitude of the signal has been amplified. This effect cannot be achieved with an Automatic Gain Control (AGC), for instance, since with it the offset would still be amplified.

Obviously, the embodiments of the invention are not restricted to the above-mentioned values and numbers of resistors and other components. For instance, the number of resistors R1 to R11 is restricted only by the number of free switch outputs 0 to 10 of the microcontroller 708.

With the measured value processing according to the invention, in particular in relation to gas sensors, more precise, temperature-independent measurements, with long term stability, are possible because of the better signal resolution. Although the special case of an NDIR CO₂ sensor is always described above, it is clear that this invention can be adapted for all sensor systems in which an analog sensor signal with offset is present.

## Claims

1. Method of processing an analog sensor signal, the method including the following steps:
feeding the analog sensor signal into a first input of an operational amplifier;
amplifying the analog sensor signal by the operational amplifier;
measuring the amplified analog sensor signal;
comparing the amplified analog sensor signal with a threshold value;
generating a direct voltage depending on a difference between the amplified analog sensor signal and the threshold value;
forming a difference signal from the analog sensor signal and the direct voltage; and
amplifying the difference signal and outputting an output signal.

2. Method according to claim 1, wherein the direct voltage is generated by a variable resistance between a second input of the operational amplifier and a reference point with a reference voltage.

3. Method according to either claim 1 or claim 2, wherein the step of generating the direct voltage includes the following step:
selecting the reference point to which the variable resistance is connected.

4. Method according to at least one of the preceding claims, wherein the reference voltage is an operating voltage or a reference potential.

5. Method according to any one of the preceding claims, wherein the reference potential is earth potential.

6. Method according to at least one of the preceding claims, wherein the step of generating the direct voltage includes the following step:
generating the variable resistance by connecting in parallel at least one other resistor in a plurality of resistors.

7. Method according to at least one of the preceding claims, wherein the step of generating the direct voltage includes the following step:
connecting the plurality of resistors by a controller, which activates at least one switch according to a predefined combination.

8. Method according to at least one of the preceding claims, wherein all resistors of the plurality of resistors have the same value.

9. Method according to any one of claims 1 to 7, wherein the resistors of the plurality of resistors have different values.

10. Method according to at least one of the preceding claims, wherein the operational amplifier has two differential amplifiers.

11. Method of processing an analog sensor signal according to at least one of the preceding claims, wherein the step of measuring the amplified analog sensor signal includes:
converting the amplified analog sensor signal into a digital signal, and analyzing the digital signal by a controller.

12. Method of processing an analog sensor signal according to claim 11, wherein the threshold value is a maximum operating voltage of the controller.

13. Method of processing an analog sensor signal according to at least one of the preceding claims, wherein the analog sensor signal is generated by a pyrosensor.

14. Method according to at least one of the preceding claims, wherein the analog sensor signal indicates the presence and/or the concentration of a polar gas, preferably carbon dioxide.

15. A measured value processing device for processing an analog sensor signal, the measured value processing device including:
an operational amplifier to amplify the analog sensor signal which was fed into a first input;
a controller (708) to measure the amplified analog sensor signal and compare the amplified analog sensor signal with a threshold value;
wherein the controller is in such a form that it generates a direct voltage depending on a difference between the amplified analog sensor signal and the threshold value; and the operational amplifier amplifies a difference signal from the analog sensor signal and the direct voltage, and outputs it as an output signal.

16. Measured value processing device according to claim 15, also including:
an analog/digital converter (702) to convert the amplified analog sensor signal into a digital signal, the controller (708) analyzing the digital signal.

17. Measured value processing device according to either claim 15 or claim 16, also including:
a variable resistor between a second input of the operational amplifier and a reference point with a reference voltage.

18. Measured value processing device according to any one of claims 15 to 17, wherein the variable resistance is generated by connecting in parallel at least one other resistor in a plurality of resistors (R1 to R11), the resistors in the plurality of resistors either all having the same value or having different values.

19. Measured value processing device according to any one of claims 15 to 18, wherein the operational amplifier has two differential amplifiers (704, 706).

20. Gas sensor arrangement (100) with at least one radiation-emitting radiation source (102), a gas measurement space (104), which can be filled with a measurement gas which includes at least one analyte to be measured, and at least one radiation-detecting detector unit (108), which generates an analog sensor signal which depends on the absence and/or concentration of the analyte, and with a measured value processing device to capture the analog sensor signal and output an amplified output signal,
wherein the measured value processing device is adapted to execute the method according to any one of claims 1 to 14.
